(19) Europäisches Patentamt
European Patent Office
Office européen des brevets

(11) Veröffentlichungsnummer: **0 308 783**
**A2**

(12) # EUROPÄISCHE PATENTANMELDUNG

(21) Anmeldenummer: **88114934.8**

(22) Anmeldetag: **13.09.88**

(51) Int. Cl.4: **A61K 31/41 , A61K 31/415 , C07D 249/08 , C07D 233/60**

(30) Priorität: **25.09.87 DE 3732386**

(43) Veröffentlichungstag der Anmeldung: **29.03.89 Patentblatt 89/13**

(84) Benannte Vertragsstaaten: **AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Stroech, Klaus, Dr.**
**Rolsberger Strasse 22**
**D-5650 Solingen 19(DE)**
Erfinder: **Böckmann, Klaus, Dr.**
**Claudiusweg 7**
**D-5600 Wuppertal 1(DE)**
Erfinder: **Plempel, Manfred, Dr.**
**Zwengenberger Strasse 3c**
**D-5657 Haan(DE)**

(54) **Antimykotische Mittel.**

(57) Die vorliegende Erfindung betrifft neue Hydroxyethylazolyl-Derivate zur Behandlung von Krankheiten, insbesondere Mykosen.

EP 0 308 783 A2

## Antimykotische Mittel

Die vorliegende Erfindung betrifft neue hydroxyethylazolyl-Derivate sowie deren pharmakologisch verträgliche Säureadditions-Salze zur Behandlung von Krankheiten, insbesondere Mykosen.

Es ist bereits bekannt geworden, daß bestimmte Azolylmethyl-cyclopropyl-carbinol-Derivate, wie beispielsweise 1-(4-Chlorphenyl)-1-(1-fluor-cycloprop-1-yl)-2-(1,2,4-triazol-1-yl)-ethan-1-ol und 1-(4-Chlorphenyl)-1-[1-(2,4-dichlorphenoxy)-cycloprop-1-yl]-2-(1,2,4-triazol-1-yl)-ethan-1-ol, antimykotische Eigenschaften aufweisen (vergleiche EP-OS 0 180 850). Die Wirkung dieser Stoffe ist jedoch nicht immer in allen Indikationsbereichen voll zufriedenstellend.

Es wurde gefunden, daß die neuen Hydroxyethyl-azolyl-Derivate der Formel

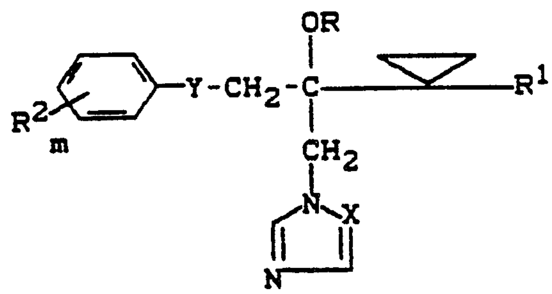

(I)

in welcher

R für Wasserstoff, Alkyl oder Acyl steht,

$R^1$ Für Halogen, gegebenenfalls substituiertes Phenyl oder die Gruppierung -Z-$R^3$ steht, worin

  Z für Sauerstoff, Schwefel, SO oder $SO_2$ steht und

  $R^3$ für gegebenenfalls substituiertes Phenyl steht,

$R^2$ für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Alkoximinomethyl mit 1 bis 4 Kohlenstoffatomen in der Alkoxygruppe, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht,

m für die Zahlen 0, 1, 2 oder 3 steht,

X für Stickstoff oder eine CH-Gruppe steht und

Y für Sauerstoff, Schwefel, SO oder $SO_2$ steht,

sowie deren Säureadditions-Salze gute antimikrobielle, insbesondere antimykotische Eigenschaften aufweisen.

Die erfindungsgemäß zu verwendenden Hydroxyethylazolyl-Derivate sind durch die Formel (I) allgemein definiert. In dieser Formel stehen vorzugsweise

R für Wasserstoff, Alkyl mit 1 bis 6 Kohlenstoffatomen oder Alkylcarbonyl mit 1 bis 6 Kohlenstoffatomen in der Alkylgruppe,

$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung -Z-$R^3$, worin

  Z für Sauerstoff, Schwefel, SO oder $SO_2$ steht und

  $R^3$ für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht,

$R^2$ für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy,

m für die Zahlen 0, 1, 2 oder 3,

X für Stickstoff oder eine CH-Gruppe und

Y für Sauerstoff, Schwefel, SO oder $SO_2$.

Wenn m für die Zahlen 2 oder 3 steht, können die für $R^2$ stehenden Reste gleich oder verschieden sein.

Besonders bevorzugt sind diejenigen Verbindungen der Formel (I), in denen

R für Wasserstoff, Methyl, Ethyl, n-Propyl, Isopropyl, n-Butyl, Isobutyl, Methylcarbonyl, Ethylcarbonyl, n-Propylcarbonyl, Isopropyl-carbonyl, n-Butylcarbonyl und Isobutyl-carbonyl steht,

$R^1$ für Fluor, Chlor, Brom, gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht oder für die Gruppierung -Z-$R^3$ steht, worin

Z für Sauerstoff, Schwefel, SO oder $SO_2$ steht und

$R^3$ für gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht,

$R^2$ für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder ethyl substituiertes Phenoxy steht,

m für die Zahlen 0, 1, 2 oder 3 steht,

X für Stickstoff oder eine CH-Gruppe steht und

Y für Sauerstoff, Schwefel, SO oder $SO_2$ steht.

Bevorzugte erfindungsgemäße Verbindungen sind auch pharmakologisch verträgliche Additionsprodukte aus Säuren und denjenigen Hydroxyethyl-azolyl-Derivaten der Formel (I), in denen R, $R^1$, $R^2$, X, Y und m die Bedeutungen haben, die bereits vorzugsweise für diese Reste bzw. diesen Index genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäure und Hydroxycarbonsäuren, wie z.B. Essigsäure, Maleinsäure, Bernsteinsäure, Fumarsäure, Weinsäure, Zitronsäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Als Beispiele für Hydroxyethyl-azolyl-Derivate der Formel (I) seien die in der folgenden Tabelle aufgeführten Stoffe genannt.

Tabelle 1

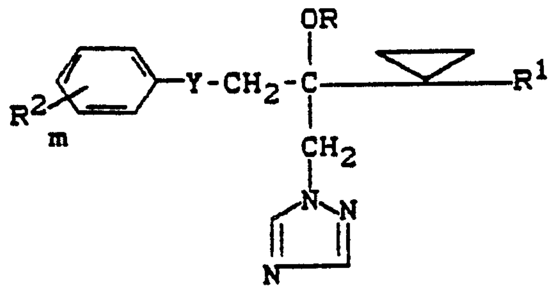

(I)

| $R^2_m$ | R | X | $R^1$ | Y |
|---|---|---|---|---|
| 2,4-$Cl_2$ | H | N | Cl | O |
| 2,4-$F_2$ | H | N | Cl | O |
| 4-$CH_3$ | H | N | Cl | O |
| 4-$CF_3$ | H | N | Cl | O |
| 4-$OCF_3$ | H | N | Cl | O |
| 4-$OCH_3$ | H | N | Cl | O |
| 4-$SCH_3$ | H | N | Cl | O |
| 2,4,6-$Cl_3$ | H | N | Cl | O |
| 4-Cl | H | N | F | O |
| 4-Cl | H | CH | Cl | O |
| 4-Cl | $CH_3$ | N | Cl | O |
| 4-Cl | H | N | 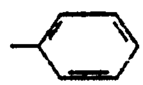 | O |
| 4-Cl | H | N | 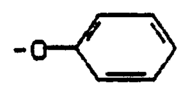 | O |
| 4-Cl | H | N | 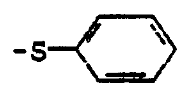 | O |
| 4-Cl | H | N | 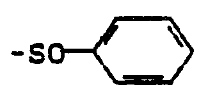 | O |
| 4-Cl | H | N | 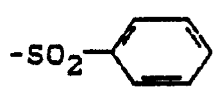 | O |
| 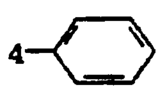 | H | N | Cl | O |

Tabelle 1 (Fortsetzung)

| $R^2{}_m$ | R | X | $R^1$ | Y |
|---|---|---|---|---|
| 4-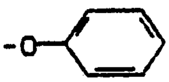 | H | N | Cl | O |
| 4-$C_4H_9$-t. | H | N | Cl | O |
| 2-Cl,4-$CH_3$ | H | N | Cl | O |
| | H | N | Cl | O |
| 4-Cl | -CO-$CH_3$ | N | Cl | O |
| 4-Cl | -$C_2H_5$ | N | Cl | O |
| 4-F | $CH_3$ | N | F | O |
| 2,4-$Cl_2$ | H | N | Cl | S |
| 2,4-$F_2$ | H | N | Cl | S |
| 4-$CH_3$ | H | N | Cl | S |
| 4-$CF_3$ | H | N | Cl | S |
| 4-$OCF_3$ | H | N | Cl | S |
| 4-$OCH_3$ | H | N | Cl | S |
| 4-$SCH_3$ | H | N | Cl | S |
| 2,4,6-$Cl_3$ | H | N | Cl | S |
| 4-Cl | H | N | F | S |
| 4-Cl | H | CH | Cl | S |
| 4-Cl | $CH_3$ | N | Cl | S |
| 4-Cl | H | N | 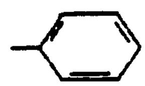 | S |
| 4-Cl | H | N | -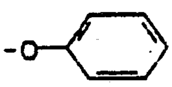 | S |
| 4-Cl | H | N | -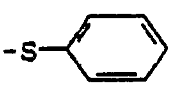 | S |
| 4-Cl | H | N | -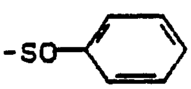 | S |
| 4-Cl | H | N | -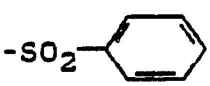 | S |
| 4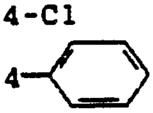 | H | N | Cl | S |

Tabelle 1 (Fortsetzung)

| $R^2_m$ | R | X | $R^1$ | Y |
|---|---|---|---|---|
| 4-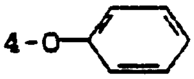 | H | N | Cl | S |
| $4-C_4H_9$-t. | H | N | Cl | S |
| $2-Cl,4-CH_3$ | H | N | Cl | S |
| - | H | N | Cl | S |
| 4-Cl | $-CO-CH_3$ | N | Cl | S |
| 4-Cl | $-C_2H_5$ | N | Cl | S |
| 4-F | $CH_3$ | N | F | S |
| $2,4-Cl_2$ | H | N | Cl | SO |
| $2,4-F_2$ | H | N | Cl | SO |
| $4-CH_3$ | H | N | Cl | SO |
| $4-CF_3$ | H | N | Cl | SO |
| $4-OCF_3$ | H | N | Cl | SO |
| $4-OCH_3$ | H | N | Cl | SO |
| $2,4,6-Cl_3$ | H | N | Cl | SO |
| 4-Cl | H | N | F | SO |
| 4-Cl | H | CH | Cl | SO |
| 4-Cl | $CH_3$ | N | Cl | SO |
| 4-Cl | H | N | 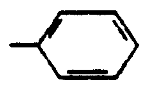 | SO |
| 4-Cl | H | N | 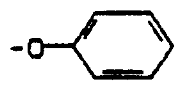 | SO |
| 4-Cl | H | N | 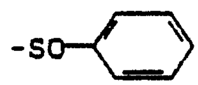 | SO |
| 4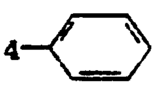 | H | N | Cl | SO |

Tabelle 1 (Fortsetzung)

| $R^2_m$ | R | X | $R^1$ | Y |
|---|---|---|---|---|
| 4-O 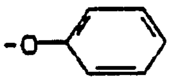 | H | N | Cl | SO |
| 4-$C_4H_9$-t. | H | N | Cl | SO |
| 2-Cl,4-$CH_3$ | H | N | Cl | SO |
| - | H | N | Cl | SO |
| 4-Cl | -CO-$CH_3$ | N | Cl | SO |
| 4-Cl | -$C_2H_5$ | N | Cl | SO |
| 4-F | $CH_3$ | N | F | SO |
| 2,4-$Cl_2$ | H | N | Cl | $SO_2$ |
| 2,4-$F_2$ | H | N | Cl | $SO_2$ |
| 4-$CH_3$ | H | N | Cl | $SO_2$ |
| 4-$CF_3$ | H | N | Cl | $SO_2$ |
| 4-$OCF_3$ | H | N | Cl | $SO_2$ |
| 4-$OCH_3$ | H | N | Cl | $SO_2$ |
| 2,4,6-$Cl_3$ | H | N | Cl | $SO_2$ |
| 4-Cl | H | N | F | $SO_2$ |
| 4-Cl | H | CH | Cl | $SO_2$ |
| 4-Cl | $CH_3$ | N | Cl | $SO_2$ |
| 4-Cl | H | N | 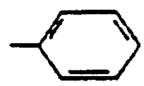 | $SO_2$ |
| 4-Cl | H | N | -O 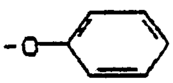 | $SO_2$ |
| 4-Cl | H | N | -$SO_2$ 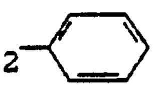 | $SO_2$ |
| 4 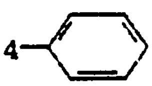 | H | N | Cl | $SO_2$ |

Tabelle 1 (Fortsetzung)

| $R^2_m$ | R | X | $R^1$ | Y |
|---|---|---|---|---|
| 4-O 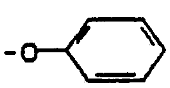 | H | N | Cl | $SO_2$ |
| 4-$C_4H_9$-t. | H | N | Cl | $SO_2$ |
| 2-Cl,4-$CH_3$ | H | N | Cl | $SO_2$ |
| - | H | N | Cl | $SO_2$ |
| 4-Cl | -CO-$CH_3$ | N | Cl | $SO_2$ |
| 4-Cl | -$C_2H_5$ | N | Cl | $SO_2$ |
| 4-F | $CH_3$ | N | F | $SO_2$ |

Die erfindungsgemäß zu verwendenden Hydroxyethyl-azolyl-Derivate der Formel (I) sowie ihre Säureadditions-Salze sind noch nicht bekannt. Sie sind jedoch Gegenstand einer Patentanmeldung, die noch nicht veröffentlicht ist (vergleiche die Deutsche Patentanmeldung P 3 721 696 vom 01.07.1987). Sie

können nach den dort beschriebenen Verfahren erhalten werden, indem man

a) Oxirane der Formel

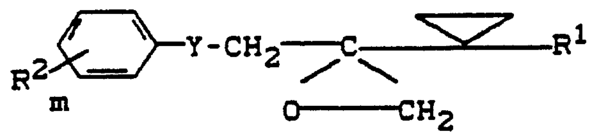

(II)

in welcher

$R^1$, $R^2$, Y und m die oben angegebene Bedeutung haben,

mit Azolen der Formel

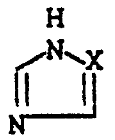

(III)

in welcher

X die oben angegebene Bedeutung hat,

in Gegenwart eines Säurebindemittels, wie beispielsweise Kaliumcarbonat und in Gegenwart eines Verdünnungsmittels, wie beispielsweise Acetonitril, bei Temperaturen zwischen 50°C und 150°C umsetzt,

oder

b) Azolyl-Derivate der Formel

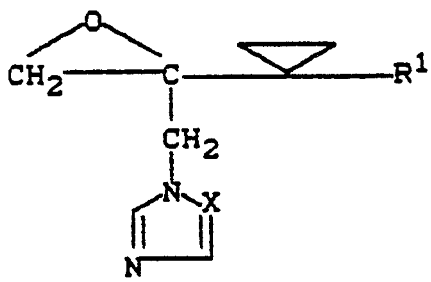

(IV)

in welcher

$R^1$ und X die oben angegebene Bedeutung haben,

mit Phenyl-Derivaten der Formel

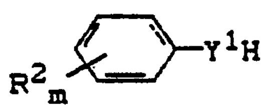

(V)

in welcher

$R^2$ und m die oben angegebene Bedeutung haben und

$Y^1$ für Sauerstoff oder Schwefel steht,

in Gegenwart eines Säurebindemittels, wie beispielsweise Kaliumhydroxid und in Gegenwart eines Verdünnungsmittels, wie beispielsweise Acetonitril, bei Temperaturen zwischen 50°C und 150°C umsetzt,

oder

c) Hydroxyethyl-azolyl-Derivate der Formel

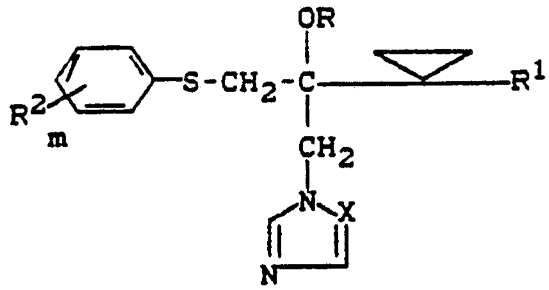

(Ia)

in welcher
R, $R^1$, $R^2$, X und m die oben angegebene Bedeutung haben,
mit Oxidationsmitteln gegebenenfalls in Gegenwart eine Verdünnungsmittels, wie beispielsweise Wasserstoffperoxid/Eisessig, bei Temperaturen zwischen -30 und +50 °C umsetzt,
oder

d) Hydroxyethyl-azolyl-Derivate der Formel

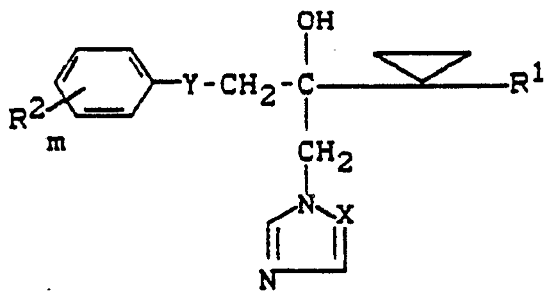

(Ib)

in welcher
$R^1$, $R^2$, X, Y und m die oben angegebene Bedeutung haben,
mit starken Basen, wie beispielsweise Alkalimetallamide oder -hydride in Gegenwart eines Verdünnungsmittels, wie beispielsweise Dioxan, bei Temperaturen zwischen 20 °C und 100 °C umsetzt und die dabei entstehenden Alkoholate der Formel

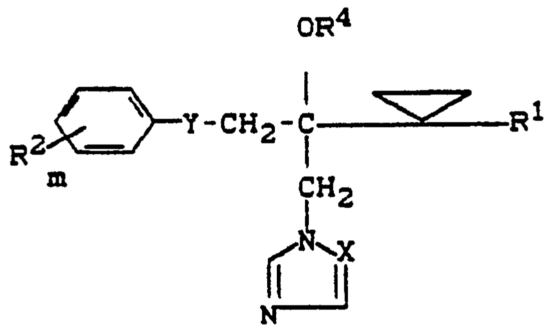

(Ic)

in welcher
$R^1$, $R^2$, X, Y und m die oben angegebene Bedeutung haben, und
$R^4$ für einen kationischen Rest einer Base steht,
mit Halogenverbindungen der Formel

$R^5$ - Hal (VI)

in welcher
$R^5$ für Alkyl oder Acyl steht und
Hal für Halogen steht,
in Gegenwart eines Verdünnungsmittels, wie beispielsweise Dioxan, bei Temperaturen zwischen 20 °C und

100 ° C umsetzt,
und gegebenenfalls anschließend an die so erhaltenen Verbindungen der Formel (I) in üblicher Weise eine Säure addiert.

Die erfindungsgemäßen Stoffe erhalten ein asymmetrisch substituiertes Kohlenstoffatom. Sie können daher in optischen Isomerenformen anfallen. Die vorliegende Erfindung betrifft sowohl die einzelnen Isomeren als auch deren Gemische.

Die für das Verfahren (a) als Ausgangsstoffe zu verwendenden Oxirane der Formel (II) sind bisher noch nicht bekannt. Sie lassen sich herstellen, indem man

e) Cyclopropylketone der Formel

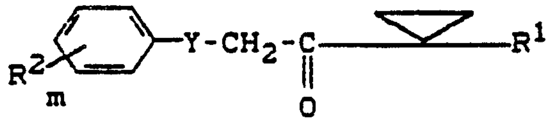
(VII)

in welcher
$R^1$, $R^2$, Y und m die oben angegebene Bedeutung haben,
entweder
α) mit Dimethyloxosulfonium-methylid der Formel

$$(CH_3)_2\overset{\delta\oplus}{S}O\overset{\delta\ominus}{C}H_2 \quad (VIII)$$

oder
β) mit Dimethylsulfonium-methylid der Formel

$$(CH_3)_2\overset{\delta\oplus}{S}\ \ \overset{\delta\ominus}{C}H_2 \quad (IX)$$

in Gegenwart eines Verdünnungsmittels umsetzt.
Die bei dem Verfahren (e) als Ausgangsstoffe benötigten Cyclopropylketone der Formel (VII) sind ebenfalls noch nicht bekannt. Sie lassen sich herstellen, indem man

f) Halogenketone der Formel

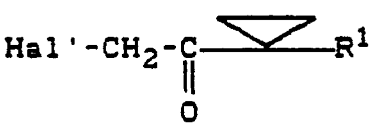
(X)

in welcher
$R^1$ die oben angegebene Bedeutung hat und
Hal′ für Chlor oder Brom steht,
mit Phenyl-Derivaten der Formel

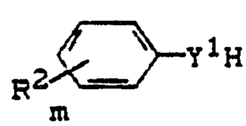
(V)

in welcher
$R^2$, $Y^1$ und m die oben angegebene Bedeutung haben,
in Gegenwart eines Säurebindemittels und gegebenenfalls in Gegenwart eines Verdünnungsmittels umsetzt und gegebenenfalls anschließend oxidiert.

Die bei dem Verfahren (f) Ausgangsstoffe benötigten Halogenketone der Formel (X) sind teilweise bekannt. Sie lassen sich herstellen, indem man

g) Ketone der Formel

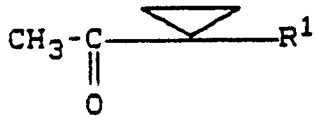 (XI)

in welcher

$R^1$ die oben angegebene Bedeutung hat,

mit Chlorierungsmitteln oder Bromierungsmitteln in Gegenwart eines Verdünnungsmittels umsetzt.

Die bei dem Verfahren (g) Ausgangsstoffe benötigten Ketone der Formel (XI) sind bekannt oder lassen sich nach prinzipiell bekannten Verfahren synthetisieren (vgl. Synthesis 1977, 189).

Als Chlorierungsmittel und Bromierungsmittel kommen bei dem Verfahren (g) alle für derartige Umsetzungen üblichen Chlorierungs- und Bromierungsreagenzien in Be tracht. Vorzugsweise verwendbar sind Sulfurylchlorid, Sulfurylbromid und Brom.

Als Verdünnungsmittel kommen bei dem Verfahren (g) alle für derartige Umsetzungen üblichen inerten organischen Solventien in Frage. Vorzugsweise verwendbar sind halogenierte aliphatische Kohlenwasserstoffe, wie Methylenchlorid, Chloroform und Tetrachlorkohlenstoff.

Die Reaktionstemperaturen können bei dem Verfahren (g) innerhalb eines bestimmten Bereiches variiert werden. Im allgemeinen arbeitet man bei Temperaturen zwischen -10 °C und +60 °C, vorzugsweise zwischen 0 °C und +40 °C.

Bei der Durchführung des Verfahrens (g) arbeitet man ebenso wie bei den übrigen in dieser Anmeldung beschrieben Verfahren im allgemeinen unter Normaldruck. Es ist jedoch jeweils auch möglich, unter erhöhtem oder vermindertem Druck zu arbeiten.

Bei der Durchführung des Verfahrens (g) setzt man auf 1 Mol an Keton der Formel (XI) im allgemeinen eine stöchiometrische Menge oder auch einen geringen Überschuß an Chlorierungs- oder Bromierungsmittel ein. Die Aufarbeitung erfolgt nach üblichen Methoden. Im allgemeinen geht man so vor, daß das Reaktionsgemisch nacheinander mit verdünnter, wäßriger Natriumhydrogencarbonat-Lösung und Wasser wäscht, dann trocknet und einengt.

Die bei dem Verfahren (f) als Reaktionskomponenten benötigten Phenyl-Derivate sind durch die Formel (V) allgemein definiert. In dieser Formel haben $R^2$, $Y^1$ und m vorzugsweise diejenigen Bedeutungen, die bereits im Zusammenhang mit der Beschreibung der erfindungsgemäßen Stoffe der Formel (I) vorzugsweise für diese Reste und für diesen Index genannt wurden. Die Phenyl-Derivate der Formel (V) sind allgemein bekannte Verbindungen der organischen Chemie.

Als Säurebindemittel kommen bei der Durchführung des Verfahrens (f) alle üblichen Säureakzeptoren in Betracht. Vorzugsweise verwendbar sind Alkalimetallcarbonate und Hydrogencarbonate, wie Natriumcarbonat, Kaliumcarbonat oder Natriumhydrogencarbonat, ferner Alkalimetallhydroxide und -Alkoholate, wie Natriumhydroxid, Kaliumhydroxid, Natriummethylat oder Kalium-tert.-butylat, außerdem tertiäre aliphatische oder aromatische Amine, wie Triethylamin, N,N-Dimethyl-cyclohexyl-amin, N,N-Dimethyl-benzylamin und Pyridin, und außerdem cylische Amine, wie 1,5-Diaza-bicyclo [4.3.0]non-5-en (DBN), 1,8-Diaza-bicyclo[5.4.0]undec-7-en (DBU) und 1,4-Diaza-bicyclo[2.2.2]octan (DABCO).

Als Verdünnungsmittel kommen bei der Durchführung des Verfahrens (f) alle inerten organischen Solventien in Betracht. Vorzugsweise verwendbar sind aliphatische und aromatische, gegebenenfalls halogenierte Kohlenwasserstoffe wie Pentan, Hexan, Heptan, Cylohexan, Petrolether, Benzin, Ligroin, Benzol, Toluol, Xylol, Methylenchlorid, Ethylenchlorid, Chloroform, Tetrachlorkohlen stoff, Chlorbenzol und o-Dichlorbenzol, Ether wie Dimethyl- und Dibutylether, Glykoldimethylether und Diglykoldimethylether, Tetrahydrofuran und Dioxan, Ketone wie Aceton, Methylethyl-, Methyl-isopropyl- und Methyl-isobutyl-keton, Ester wie Essigsäuremethylester und -ethylester, Nitrile wie z.B. Acetonitril und Propionitril und Pyridin, sowie auch stark polare Lösungsmittel, wie Dimethylformamid und Dimethylsulfoxid.

Die Reaktionstemperaturen können bei dem Verfahren (f) innerhalb eines größeren Bereichs variiert werden. Im allgemeinen arbeitet man bei Temperaturen zwischen 0 °C und 150 °C, vorzugsweise zwischen 20 °C und 130 °C.

Bei der Durchführung des Verfahrens (f) setzt man auf 1 Mol an Halogenketon der Formel (X) im

allgemeinen 1 bis 1,5 an Phenyl-Derivat der Formel (V) sowie 1 bis 2 Mol an Säurebindemittel ein. Die Aufarbeitung erfolgt nach üblichen Methoden. Im allgemeinen geht man so vor, daß man gegebenenfalls nach vorherigem Abfiltrieren von abgeschiedenen Salzen das Reaktionsgemisch einengt, den Rückstand in einem mit Wasser wenig mischbaren organischen Lösungsmittel aufnimmt, die entstehende Lösung wäscht, trocknet und dann einengt.

Das bei dem Verfahren (e) als Reaktionskomponente benötigte Dimethyl-oxo-sulfonium-methylid der Formel (VIII) ist bekannt (vgl. J. Am. Chem. soc. 87, 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid oder Natriumamid, Kalium-tert.-butylat oder Natriummethylat, in Gegenwart eines Verdünnungsmittels erzeugt.

Das bei dem Verfahren (e) außerdem als Reaktionskomponente in Betracht kommende Dimethylsulfonium-methylid der Formel (IX) ist ebenfalls bekannt (vgl. Heterocycles 8, 397 (1977)). Es wird bei der obigen Umsetzung ebenfalls in frisch hergestelltem Zustand eingesetzt, indem man es in situ zum Beispiel aus Trimethylsulfoniumhalogenid oder Trimethylsulfoniummethylsulfat, in Gegenwart einer starken Base, wie zum Beispiel Natriumhydrid, Natriumamid, Natriummethylat, Kalium-tert.-butylat oder Kaliumhydroxid, in Gegenwart eines Verdünnungsmittels, wie tert.-Butanol oder Dimethylsulfoxid erzeugt.

Als Verdünnungsmittel kommen bei der Durchführung des Verfahrens (e) inerte organische Solventien in Frage. Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol, sowie stark polare Lösungsmittel, wie Dimethylsulfoxid oder Acetonitril.

Die Reaktionstemperaturen können bei der Durchführung des Verfahrens (e) in einem größeren Bereich variiert werden. Im allgemeinen arbeitet man zwischen 0 °C und 100 °C, vorzugsweise zwischen 10 °C und 60 °C.

Bei der Durchführung des Verfahrens (e) setzt man auf 1 Mol an Cyclopropyl-keton der Formel (VII) im allge meinen 1 bis 3 Mol an Dimethyloxosulfonium-methylid der Formel (VIII) bzw. an Dimethylsulfonium-methylid der Formel (IX) ein. Die Isolierung der Oxirane der Formel (II) erfolgt nach üblichen Methoden.

Die für das Verfahren (b) als Ausgangsstoffe zu verwendenden Azolyl-Derivate der Formel (IV) sind bisher noch nicht bekannt. Sie lassen sich herstellen, indem man

h) Azolylketone der Formel

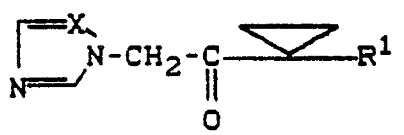

(XII)

in welcher
$R^1$ und X die oben angegebene Bedeutung haben,
entweder
α) mit Dimethyloxosulfonium-methylid der Formel

$$\overset{\delta\oplus}{(CH_3)_2SO}\ \overset{\delta\ominus}{CH_2} \qquad (VIII)$$

oder
β) mit Dimethylsulfonium-methylid der Formel

$$\overset{\delta\oplus}{(CH_3)_2S}\ \overset{\delta\ominus}{CH_2} \qquad (IX)$$

in Gegenwart eines Verdünnungsmittels umsetzt.

Die bei der Durchführung des Verfahrens (h) als Ausgangsstoffe benötigten Azolylketone sind ebenfalls noch nicht bekannt. Sie lassen sich herstellen, indem man

i) Halogenketone der Formel

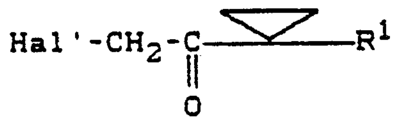

(X)

in welcher
$R^1$ die oben angegebene Bedeutung hat und
Hal′ für Chlor oder Brom steht,
mit Azolen der Formel

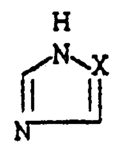

(III)

in welcher
X die oben angegebene Bedeutung hat,
in Gegenwart eines Säurebindemittels und in Gegenwart eines Verdünnungsmittels umsetzt.

Bei der Durchführung des Verfahrens (i) entsprechen die Reaktionsbedingungen denjenigen des Verfahrens (f).

Die Durchführung des Verfahrens (h) erfolgt analog zu derjenigen des Verfahrens (e).

Die außerdem bei der Durchführung des erfindungsgemäßen Verfahrens (b) als Reaktionskomponenten benötigten Phenyl-Derivate wurden bereits im Zusammenhang mit der Beschreibung des Verfahrens (f) abgehandelt.

Die für das erfindungsgemäße Verfahren (c) als Ausgangsstoffe benötigten Hydroxyethyl-azolyl-Derivate der Formel (Ia) sind erfindungsgemäße Verbindungen.

. Als Reaktionskomponenten kommen bei dem erfindungsgemäßen Verfahren (c) alle für derartige Umsetzungen üblichen Oxidationsmittel in Betracht. Vorzugsweise verwendbar sind Wasserstoffperoxid und Persäuren, wie m-Chlorperbenzoesäure und Peressigsäure.

Bei der Durchführung des erfindungsgemäßen Verfahrens (c) setzt man auf 1 Mol der erfindungsgemäßen Verbindungen der Formel (Ia) etwa 1 bis 5 Mol Oxidationsmittel ein. Bei der Anwendung von 1 Mol Oxidationsmittel, wie m-Chlorperbenzoesäure in Methylenchlorid oder Wasserstoffperoxid in Essigsäure oder Acetanhydrid bei Temperaturen zwischen -30 °C bis +90 °C, entstehen vorzugsweise die erfindungsgemäßen Verbindungen der Formel (I), in denen Y for eine -SO-Gruppierung steht. Bei Überschuß an Oxidationsmittel und Temperaturen zwischen 10 °C und 90 °C entstehen vorzugsweise die erfindungsgemäßen Verbindungen der Formel (I), in denen Y for eine -$SO_2$-Gruppierung steht. Die Isolierung der Oxidationsprodukte erfolgt in üblicher Weise.

Die für das erfindungsgemäße Verfahren (d) aus Ausgangsstoffe benötigten Hydroxyethyl-azolyl-Derivate der Formel (Ib) ebenfalls erfindungsgemäße Verbindungen. Ihre Überführung in die entsprechenden Alkoholate erfolgt in allgemein bekannter Weise, indem man mit geeigneten starken Basen, wie Alkalimetall-amiden oder -hydriden, quarternären Ammonium-hydroxiden oder Phosphonium-hydroxiden in einem inerten Verdünnungsmittel, wie zum Beispiel Dioxan, bei Raumtemperatur umsetzt. Demgemäß steht $R^3$ in den Verbindungen der Formel (Ic) vorzugsweise für ein Alkalimetallkation, wie ein Natrium-oder Kalium-kation, oder für ein quarternäres Ammonium-oder Phosphonium-kation.

Die außerdem für das Verfahren (d) als Ausgangsstoffe zu verwendenden Halogenverbindungen der Formel (VI) sind bekannt oder lassen sich nach im Prinzip bekannten Methoden herstellen.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B., durch Abfiltrieren, isoliert und gegebenenfalls durch Waschen mit einem inerten organischen Lösungsmittel gereinigt werden.

Die erfindungsgemäße Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton

mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:
Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiet in der Tiermedizin können beispielsweise aufgeführt werden:
Alle Dermatomykosen und Systemmykosen, insbesondere solche, die durch die obengenannten Erreger hervorgerufen werden.

Zur vorliegenden Erfindungen gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einen Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulat, Suppositorien, Lösungen, Suspensionen und Emulsionen, Pasten, Salben, Gele, Cremes, Lotions, Puder oder Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure re, (b) Bindemittel, z.B. Carboxymethylcellulose, Alginate, Gelatine, Polyvinylpyrrolidon, (c) Feuchthaltemittel, z.B. Glycerin, (d) Sprengmittel z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Den Tabletten, Dragees, Kapseln, Pillen und Granulaten können mit den üblichen gegebenenfalls Opakisierungsmittel enthaltenen Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse ver wendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Träger stoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silocone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnüßöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B, ethoxylierte Isostearylalkohole, Polyoxyethylensorbit- und Sorbitanester, mikrokristalline Cellulose, Aluminimmetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben angeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungs gemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise 5 bis 150 mg/kg Körpergewicht je 24 Stunden, gegebenenfalls in Form mehrerer Einzelgaben zur Erzielung der gewünschten Ergebnisse zu verabreichen.

Bei oralen Applikationen werden die erfindungsgemäßen Wirkstoffe in Gesamtmengen von etwa 2,5 etwa 200, vorzugsweise 5 bis 150 mg/kg Körpergewicht je 24 Stunden und bei parenteraler Applikation in Gesamtmengen von etwa 2,5 bis etwa 50, vorzugsweise 1 bis 25 mg/kg Körpergewicht je 24 Stunden verabreicht.

Es kann jedoch erforderlich sein, von den genannten Dosierungen abzuweichen und zwar in Abhängigkeit von der Art und dem Körpergewicht des zu behandelnden Objektes, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele

Beispiel 1

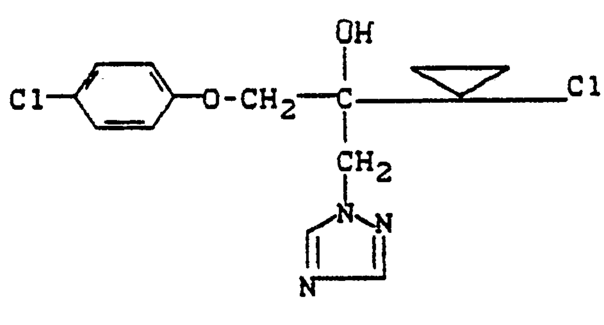

(I-1)

In ein Gemisch aus 29 g (0,42 Mol) 1,2,4-Triazol, 19 g (0,14 Mol) Kaliumcarbonat und 100 ml Acetonitril wird unter Stickstoffatmosphäre eine Lösung 27,9 g (0,11 Mol) 2-(1-Chlorcyclopropyl)-2-(4-chlor-phenoxymethyl)-oxiran in 50 ml Acetonitril eingetropft, wobei das Reaktionsgemisch unter Rückfluß gekocht wird. Nach beendeter Zugabe wird noch weitere 8 Stunden unter Rückfluß erhitzt. Anschließend saugt man vom festen Rückstand ab und engt das Filtrat unter vermindertem Druck ein. Der verbleibende Rückstand wird in

Essigester aufgenommen. Die dabei entstehende Lösung wird mit Wasser gewaschen, über Natriumsulfat getrocknet und unter vermindertem Druck eingeengt. Das so erhaltene Produkt wird auf chromatographischem Wege über Kieselgel mit Chloroform/Ethanol = 97:3 als Laufmittel gereinigt. Man erhält auf diese Weise 13,7 g (39% der Theorie) an 2-(1-Chlorcyclopropyl)-1-(4-chlorphenoxy)-3-(1,2,4-triazol-1-yl)-propan-2-ol in Form eine Öles.
$^1$H-NMR (80 MHz, $CDCl_3$):
$\delta$ = 0,5-1-4(m,4H), 4,1-4,4(m,3H), 4,75 (s,2H), 6,8-7,25 (m, 4H), 8,0 (s, 1H), 8,17 (s, 1H).

Herstellung von Ausgangsprodukten

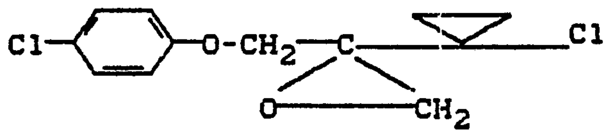

(II-1)

In ein Gemisch aus 3,9 g (0,13 Mol) Natriumhydrid (80%-ig) und 27 g (0,12 Mol) Trimethyloxosulfoniumiodid werden bei 10 °C unter Stickstoffatomosphäre und unter Rühren 90 ml absolutes Dimethylsulfoxid zugetropft. Man rührt noch 1 Stunde bei Raumtemperatur, kühlt dann auf 10 °C ab und tropft eine Lösung von 27 g (0,11 Mol) 1-Chlorcyclopropyl-4-chlorphenoxymethyl-keton in 40 ml absolutem Dimethylsulfoxid hinzu. Das Reaktionsgemisch wird zunächst 48 Stunden bei 20 °C gerührt, dann 1 Stunde auf 40 °C erwärmt und danach auf Wasser gegossen. Das entstehende Gemisch wird mit Essigester extrahiert. Die organische Phase wird mit Wasser gewaschen, über Natriumsulfat getrocknet und unter vermindertem Druck eingeengt. Man erhält auf diese Weise 27,9 g (98% der Theorie) an 2-(1-Chlorcyclopropyl)-2-(4-chlorphenoxymethyl)-oxiran in Form eines Öles.
$^1$H-NMR (60 MHz, $CDCl_3$):
$\delta$ = 0,75 - 1,4 (m, 4H), 2,8 (d, 1H), 3,05 (d, 1H), 4,25 (d, 1H), 4,58 (d, 1H), 6,8 - 7,4 (m, 4H).

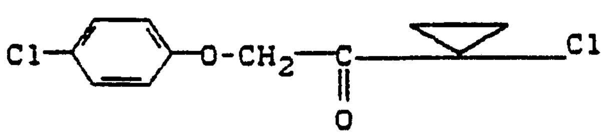

(VII-1)

In eine Lösung von 51 g (0,33 Mol) 1-Chlor-1-chloracetyl-cyclopropan in 250 ml Acetonitril werden nacheinander 49 g (0,38 Mol) 4-Chlorphenol und 70 g (0,51 Mol) Kaliumcarbonat gegeben. Das Gemisch wird 8 Stunden unter Rückfluß erhitzt, dann filtriert und eingeengt durch Abziehen des Lösungsmittels. Man nimmt den Rückstand in Essigester auf, wäscht nacheinander mit verdünnter, wäßriger Natronlauge und Wasser, trocknet über Natriumsulfat und zieht unter vermindertem Druck das Lösungsmittel ab. Der Rückstand wird einer Destillation unterworfen. Man erhält auf diese Weise 27 g (33% der Theorie) an 1-Chlor-cyclopropyl-4-chlorphenoxy-methylketon in Form einer Flüssigkeit vom Siedepunkt 125 -127 °C / 0,1 mbar.

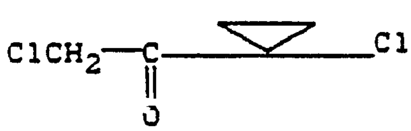

(X-1)

Bei Raumtemperatur werden 40,5 ml (0,5 Mol) Sulfurylchlorid unter Rühren langsam in eine Lösung von 54 g (0,46 Mol) 1-Acetyl-1-chlor-cyclopropan in 250 ml Methylenchlorid eingetropft. Es wird zunächst 14 Stunden bei Raumtemperatur und dann 30 Minuten bei 30 °C gerührt. Das Reaktionsgemisch wird dann nacheinander mit gesättigter, wäßriger Natriumhydrogencarbonat-Lösung und mit Wasser gewaschen. Danach wird die organische Phase über Natriumsulfat getrocknet und unter vermindertem Druck eingeengt. Man erhält auf diese Weise 51,5 g (74% der Theorie) an 1-Chlor-2-chloracetylcyclopropan in Form einer öligen Substanz.
$^1$H-NMR (60 MHz, $CDCl_3$):
$\delta$ = 1,2 - 1,9 (m, 4H); 4,8 (s, 2H)

Beispiel 2

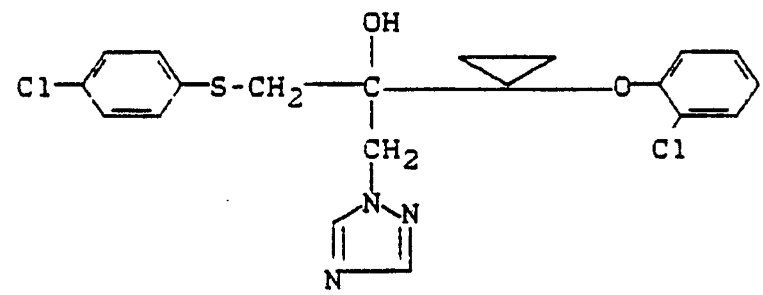

(I-2)

Ein Gemisch aus 15 g (0,05 Mol) 2-[1-(2-Chlorphenoxy)-cyclopropyl]-2-[(1,2,4-triazol-1-yl)-methyl]-oxiran, 7,5 (0,05 Mol) 4-Chlor-thiophenol, 0,75 g (0,13 Mol) Kaliumhydroxid-Pulver und 45 ml Acetonitril wird unter Rückfluß erhitzt. Man filtriert das Reaktionsgemisch und engt das Filtrat durch Abziehen des Lösungsmittels unter vermindertem Druck ein. Das dabei verbleibende Produkt wird einer chromatographischen Reinigung unterworfen. Man erhält auf diese Weise 22 g (98% der Theorie) an 1- (4-Chlorphenylmercapto)-2-[1-(2-chlorphenoxy)-cyclopropyl]-3-(1,2,4-triazol-1-yl)-propan-2-ol in Form eines öligen Produktes.
$^1$H-NMR (80 MHz, $CDCl_3$):
$\delta$ = 0,3-1,3 (m, 4H), 3,35 (d, 1H) 3,58 (d, 1H), 4,30 (S, 1H), 4,77 (S, 2H), 7,0-7,5 (m, 8H), 8,08 (S, 1H), 8,5 (S, 1H).

Herstellung von Ausgangssubstanzen:

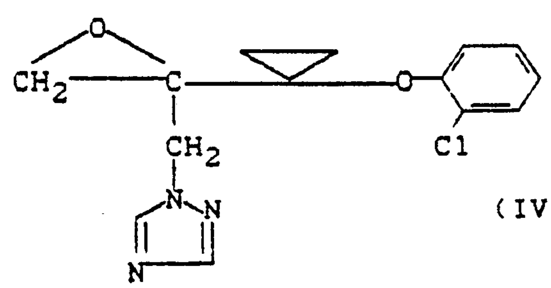

(IV-1)

In ein Gemisch aus 6 g (0,2 Mol) Natriumhydrid (80%-ig) und 42 g (0,19 Mol) Trimethyloxosulfoniumiodid werden bei 10° C unter Stickstoffatmosphäre und unter Rühren 135 ml absolutes Dimethylsulfoxid eingetropft. Das Reaktionsgemisch wird 1 Stunde bei Raumtemperatur gerührt, dann auf 10° C abgekühlt und tropfenweise mit einer Lösung von 47 g (0,17 Mol) 1-(2-Chlorphenoxy)-cyclopropyl-(1,2,4-triazol-1-yl)-methyl-keton in 50 ml absolutem Dimethylsulfoxid versetzt. Man rührt weitere 48 Stunden bei Raumtemperatur und erwärmt dann 1 Stunde auf 40° C. Man gießt danach das Reaktionsgemisch auf Wasser und extrahiert mit Essigester. Die vereinigten organischen Phasen werden mit Wasser gewaschen und nach dem Trocknen über Natriumsulfat durch Abziehen des Lösungsmittels unter vermindertem Druck eingeengt. Man erhält auf diese Weise 40,2 g (81% der Theorie) an 2-[1-(2-Chlorphenoxy)-cyclopropyl]-2-[1,2,4-triazol-1-yl)-methyl]oxiran in Form eines öligen Produktes.
$^1$H-NMR (60 MHz, $CDCl_3$):
$\delta$ = 0,6 - 1,6 (m, 4H), 2,6 (d, 1H), 2,78 (d, 1H), 4,34 (d, 1H), 4,75 (d, 1H), 6,9 - 7,5 (m, 4H), 8,0 (S, 1H), 8,45 (S, 1H).

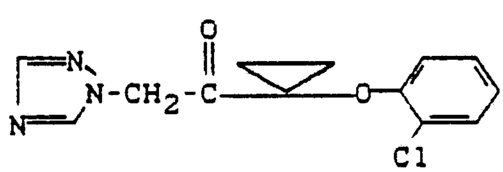

(XII-1)

In ein Gemisch aus 38,5 g (0,28 Mol) Kaliumcarbonat, 58,4 g (0,85 Mol) 1,2,4-Triazol und 140 ml Acetonitril wird eine Lösung von 62 g (0,25 Mol) 1-Chloracetyl-1-(2-chlorphenoxy)-cyclopropan in 60 ml Acetonitril eingetropft, wobei das Reaktionsgemisch unter Rückfluß gekocht wird. Das Reaktionsgemisch wird weitere 8 Stunden unter Rückfluß erhitzt, dann filtriert und durch Abziehen des Lösungsmittels unter vermindertem Druck eingeengt. Man nimmt den verbleibenden Rückstand in Essigester auf, wäscht mit Wasser, trocknet über Natrium sulfat und zieht dann das Lösungsmittel unter vermindertem Druck ab. Das verbleibende Produkt wird mit Chloroform/Ethanol = 99:1 als Laufmittel an Kieselgel chromatographiert. Man erhält auf diese Weise 47 g (67% der Theorie) an 1-(2-Chlorphenoxy)-cyclopropyl-(1,2,4-triazol-1-yl)-methylketon in Form eines öligen Produktes.

$^1$H-NMR (60 MHz, $CDCl_3$):

$\delta$ = 1,15-1,85 (m, 4H), 5,34 (S, 2H), 6,80-7,60 (m, 4H), 7,90 (S, 1H), 8,07 (S, 1H).

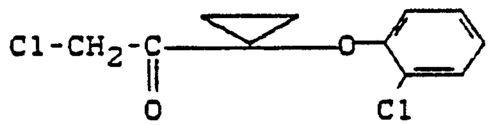

(X-2)

In ein Gemisch aus 61,7 g (0,29 Mol) 1-Acetyl-1-(2-chlorphenoxy)-cyclopropan und 0,4 g Dibenzoylperoxid werden bei 20 °C unter Rühren und unter Kühlung 48 g (0,35 Mol) Sulfurylchlorid eingetropft. Man rührt weitere 4 Stunden bei 20 °C und belichtet das Reaktionsgemisch gleichzeitig mit einer 200 W-Glühbirne. Anschließend wird das Reaktionsgemisch mit einem Gemisch aus Essigester und Toluol versetzt. Es wird mit Wasser gewaschen, über Natriumsulfat getrocknet und durch Abziehen des Lösungsmittels unter vermindertem Druck eingeengt. Das verbleibende Produkt wird einer Vakuumdestillation unterworfen. Man erhält auf diese Weise 62,3 g (87% der Theorie) an 1-Chloracetyl-1-(2-chlorphenoxy)-cyclopropan.

Kp = 117 - 122 °C / 0,2 mbar

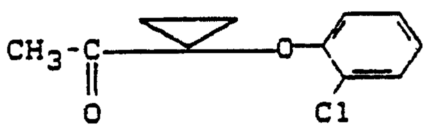

(XI-1)

In ein Gemisch aus 100 g (0,83 Mol) 5-Chlorpentan-2-on und 400 ml Methylenchlorid wird bei 10 °C unter Rühren eine Lösung von 134 g (0,84 Mol) Brom in 130 ml Methylenchlorid eingetropft. Nach beendeter Zugabe wird das Reaktionsgemisch noch 15 Minuten nachgerührt und dann auf 500 ml Wasser gegossen. Die organische Phase wird abgetrennt, nacheinander mit 5%-iger wäßriger Natriumcarbonat-Lösung und mit Wasser gewaschen, dann über Natriumsulfat getrocknet und durch Abziehen des Lösungsmittels unter vermindertem Druck eingeengt. Der Rückstand wird in 200 ml Dimethylformamid aufgenommen, und die entstehende Lösung wird bei 60 °C in ein Gemisch aus 230 g Kaliumcarbonat, 106 g (0,83 Mol) 2-Chlorphenol und 400 ml Dimethylformamid eingetropft. Das Reaktionsgemisch wird zunächst 4 Stunden bei 60 °C und dann 4 Stunden bei 90 °C gerührt. Danach saugt man vom festen Rückstand ab, zieht das Lösungsmittel unter vermindertem Druck ab und nimmt den verbleibenden Rückstand in einem Gemisch aus Essigester und Toluol auf. Die organische Phase wird mit Wasser gewaschen, über Natriumsulfat getrocknet und durch Abziehen des Lösungsmittels unter vermindertem Druck eingeengt. Der verbleibende Rückstand wird einer Vakuumdestillation unterworfen. Man erhält auf diese Weise 92,5 g (53% der Theorie) an 1-Acetyl-1-(2-chlcrphenoxy)-cyclopropan in Form einer Flüssigkeit vom Siedepunkt 81 °C / 0,2 mbar.

## Beispiel 3

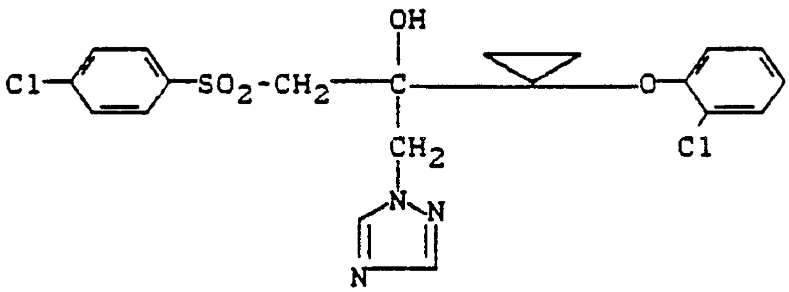

(I-3)

Eine Lösung von 10 g (23 mMol) 1-(4-Chlorphenylmercapto)-2-[1-(2-chlorphenoxy)-cyclopropyl]-3-(1,2,4-triazol-1-yl)-propan-2-ol in 50 ml Eisessig wird auf 80° C erwärmt und unter Rühren tropfenweise mit 20 ml 35%-iger Wasserstoffperoxid-Lösung versetzt. Das Reaktionsgemisch wird noch 4 Stunden auf 90° C erwärmt und dann auf Wasser gegossen. Man saugt das feste Produkt ab, nimmt es in Methylenchlorid auf, wäscht naheinander mit verdünnter, wäßriger Natronlauge und Wasser, trocknet über Natriumsulfat und engt durch Abziehen des Lösungsmittels unter vermindertem Druck ein. Man erhält auf diese Weise 6,4 g (60% der Theorie) an 1-(4-Chlorphenylsulfonyl)-2-[1-(2-chlorphenoxy)-cyclopropyl]-3-(1,2,4-triazol-1-yl)-propan-2-ol.

$^1$H-NMR (200 MHz, $CDCl_3$):

$\delta$ = 0,30 - 0,95 (m, 4H), 3,65 (d, 1H), 3,87 (d, 1H), 4,85 (d, 1H), 5,11 (d, 1H), 7,0-7,4 (m, 4H), 7,55 (d, 2H), 7,80 (d, 2H), 8,0 (s, 1H), 8,44 (s, 1H).

Nach den in den Beispielen 1 bis 3 und den in der Beschreibung angegebenen Methoden werden auch die in der folgenden Tabelle 2 formelmäßig aufgeführten Stoffe hergestellt.

Tabelle 2

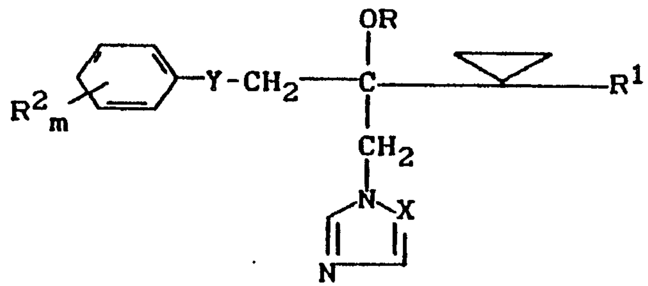

| Bsp. Nr. | Verbindung Nr. | $R^2_m$ | R | $R^1$ | Y | X | Schmelzpunkt (°C) |
|---|---|---|---|---|---|---|---|
| 4 | I-4 | 4-F | H | Cl | O | N | *) |
| 5 | I-5 | 2,4-$Cl_2$ | H | 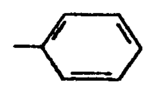 | O | N | 123 |
| 6 | I-6 | 2-$CH_3$,4-Cl | H | 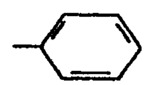 | O | N | 125 |
| 7 | I-7 | 4-CH=N-$OCH_3$ | H | 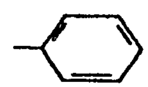 | O | N | 88 |
| 8 | I-8 | 4-Cl | H | 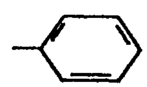 | O | N | 140 |
| 9 | I-9 | 4-Cl | H | 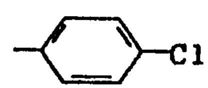 | O | N | Harz |

<u>Tabelle 2</u> (Fortsetzung)

| Bsp. Nr. | Verbindung Nr. | $R^2_m$ | R | $R^1$ | Y | X | Schmelzpunkt (°C) |
|---|---|---|---|---|---|---|---|
| 10 | I-10 | 2,4-$Cl_2$ | H | 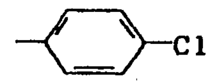 | O | N | Harz |
| 11 | I-11 | 4-$OCF_3$ | H | 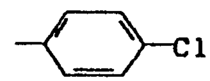 | O | N | Harz |
| 12 | I-12 | 2-$CH_3$,4-Cl | H | 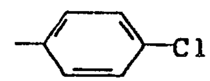 | O | N | 140 |
| 13 | I-13 | 4-CH=N-$OCH_3$ | H | 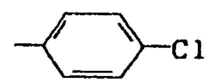 | O | N | 113 |
| 14 | I-14 | 4-F | H | 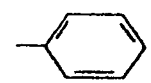 | O | N | 142 |
| 15 | I-15 | $CF_3$ | H | 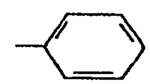 | O | N | 158 |
| 16 | I-16 | 4-$CH_3$ | H | 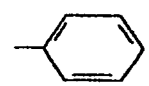 | O | N | 137 |
| 17 | I-17 | 4-$OCF_3$ | H | 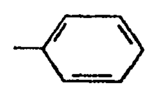 | O | N | 148 |

<u>Tabelle 2</u> (Fortsetzung)

| Bsp. Nr. | Verbindung Nr. | $R^2_m$ | R | $R^1$ | Y | X | Schmelzpunkt (°C) |
|---|---|---|---|---|---|---|---|
| 18 | I-18 | 4-$CF_3$ | H | 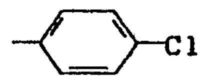 | O | N | 127 |
| 19 | I-19 | 4-$CH_3$ | H | 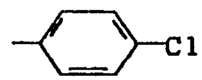 | O | N | Harz |
| 20 | I-20 | 2-Cl | H | 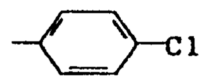 | O | N | Harz |
| 21 | I-21 | 4-F | H | 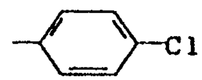 | O | N | 124 |
| 22 | I-22 | 2-Cl | H | 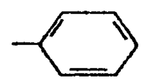 | O | N | 136 |
| 23 | I-23 | 2-$CH_3$,4-Cl | H | 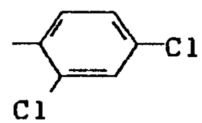 | O | N | Harz |
| 24 | I-24 | 4-Cl | H | 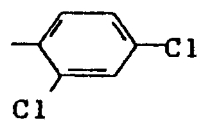 | O | N | 118 |
| 25 | I-25 | 4-CH=N-$OCH_3$ | H | 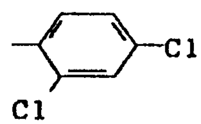 | O | N | Harz |

Tabelle 2 (Fortsetzung)

| Bsp. Nr. | Verbindung Nr. | $R^2_m$ | R | $R^1$ | Y | X | Schmelzpunkt (°C) |
|---|---|---|---|---|---|---|---|
| 26 | I-26 | 2,4-$Cl_2$ | H | 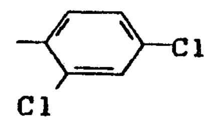 | O | N | Harz |
| 27 | I-27 | 2-Cl,4-$OCF_3$ | H | 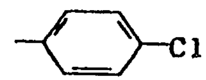 | O | N | 113 |
| 28 | I-28 | 4-$OCF_3$ | H | 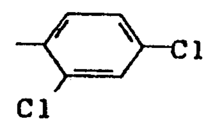 | O | N | 85 |
| 29 | I-29 | 4-Cl | H | 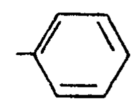 | S | N | 108 |

*) Die im Beispiel 4 offenbarte Verbindung ist durch das $^1H$-NMR-Spektrum (360 MHz, $CDCl_3$) charakterisiert:

= 0,55 - 0,70 (m, 2H), 0,80 - 0,95 (m, 1H), 1,10 - 1,20 (m, 1H) 4,10 (s, 1H), 4,16 (d, 1H), 4,30 (d, 1H), 4,75 (d, 1H), 4,80 (d, 1H), 6,80 - 7,05 (m, 4H), 8.02 (s, 1H), 8,30 (s, 1H).

Verwendungsbeispiele

In dem folgenden in-vitro-Test werden die nachstehend angegebenen Verbindungen als Vergleichssubstanzen eingesetzt:

(A) 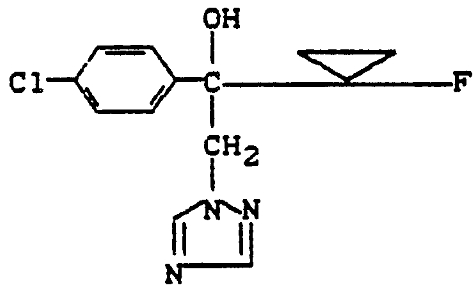

(B) 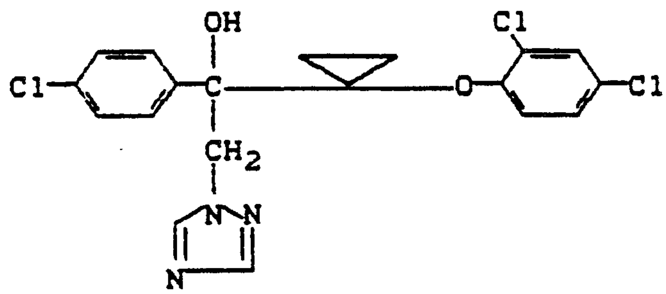

(bekannt aus EP-OS 0 180 850)

Beispiel A

Antimykotische in-vitro-Wirksamkeit

Versuchsbeschreibung:

Die in-vitro-Prüfungen wurden im Reihenverdünnungstest mit Keiminokula von durchschnittlich 5 x $10^3$ bis $10^4$ Keimen/ml Substrat durchgeführt. Als Nährmedium dienten

a) für Dermatophyten und Schimmelpilze:
Sabouraud's milieu d'epreuve

b) für Hefen:
Fleischextrakt-Traubenzucker-Bouillon.

Die Bebrütungstemperatur betrug 28 °C bis 37 °C, die Bebrütungsdauer lag bei 24 bis 96 Stunden bei Hefen und 96 Stunden bei Dermatophyten und Schimmelpilzen.

In diesem Test zeigt die erfindungsgemäße Verbindung eine bessere antimykotische Wirksamkeit als die aus dem Stand der Technik bekannten Verbindungen (A) und (B).

Tabelle A

| Antimykotische in-vitro-Wirksamkeit | | | | | |
|---|---|---|---|---|---|
| Wirkstoff | MHK-Werte in µg/ml Närmedium | | | | |
| | Trichophyton mentagr. | Microsporum canis | Candida albicans | Torulopsis glabrata | Aspergillus fumigatus |
| (A)(bekannt) | >64 | - | >64 | - | >64 |
| (B)(bekannt) | 64 | - | 16 | 64 | >64 |
| Verbindungen gemäß Herstelungsbeispiel | | | | | |
| 1 | <1 | <1 | 16 | 16 | 2 |

Beispiel B / Formulierungen

1.) Lösung:

| | |
|---|---|
| Wirkstoff gemäß Formel (I): | 10 g |
| Alkohol, rein (96 %ig) : | 300 g |
| Isopropylmyristat : | 526 g |
| | 836 g |

2.) Creme:

| | |
|---|---|
| Wirkstoff gemäß Formel (I): | 10 g |
| Aralcel 60 (Sorbitan-monostearat) : | 20 g |
| Tween 60 (Polyoxyethylen (2) -sorbitanmonostearat) : | 15 g |
| Walrat, künstlich (Mischung vom Estern von gesättigten Fettsäuren $C_{14}$-$C_{18}$ und Fettalkoholen $C_{14}$-$C_{18}$) : | 30 g |
| Lanette O : | 100 g |
| Eutanol G (2-Octyl-dodecanol) : | 135 g |
| Benzylalkohol : | 10 g |
| Wasser, entmineralisiert : | 680 g |
| | 1000 g |

## Ansprüche

1. Hydroxyethylazolyl-Derivate der allgemeinen Formel

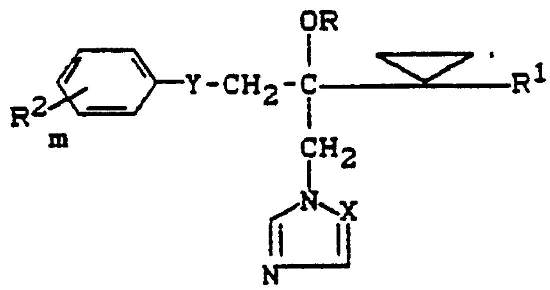

in welcher
R für Wasserstoff, Alkyl oder Acyl steht,
$R^1$ für Halogen, gegebenenfalls substituiertes Phenyl oder die Gruppierung $-Z-R^3$ steht,
worin
Z für Sauerstoff, Schwefel, SO oder $SO_2$ steht und
$R^3$ für gegebenenfalls substituiertes Phenyl steht.
$R^2$ für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis Halogenatomen, Halogenalkylthio mit 1 oder 2 Kohlenstoffatomen und 1 bis 5 Halogenatomen, Alkoximinomethyl mit 1 bis 4 Kohlenstoffatomen in der Alkoxygruppe D gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht,
m für die Zahlen 0, 1 , 2 oder 3 steht,
X für Stickstoff oder eine CH-Gruppe steht und
Y für Sauerstoff, Schwefel, SO oder $SO_2$ steht,
sowie deren Säureadditions-Salze zur Bekämpfung von Krankheiten,

2. Hydroxyethylazoyly-Derivate nach Anspruch 1,
in denen
R für Wasserstoff, Alkyl mit 1 bis 6 Kohlenstoffatomen oder Alkylcarbonyl mit 1 bis 6 Kohlenstoffatomen in der Alkylgruppe,
$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung $-Z-R^3$,
worin
Z für Sauerstoff, Schwefel, SO oder $SO_2$ steht und
$R^3$ für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht,
$R^2$ für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy,
m für die Zahlen 0, 1, 2 oder 3,
X für Stickstoff oder eine CH-Gruppe und
Y für Sauerstoff, Schwefel, SO oder $SO_2$ steht
sowie deren Säureadditions-Salze zur Bekämpfung von Krankheiten.

3. Hydroxyethylazolyl-Derivate nach Anspruch 1
in denen
R für Wasserstoff, Methyl, Ethyl, n-Propyl, Isopropyl, n-Butyl, Isobutyl, Methylcarbonyl, Ethylcarbonyl, n-Propylcarbonyl, Isopropylcarbonyl, n-Butylcarbonyl und Isobutylcarbonyl steht,
$R^1$ für Fluor, Chlor, Brom, gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht oder für die Gruppierung $-Z-R^3$ steht, worin
Z für Sauerstoff, Schwefel, SO oder $SO_2$ steht und

$R^3$ für gegebenenfalls einfach bis dreifach, gleichartig oder verschieden durch Fluor, Chlor, Brom, Trifluormethyl, Methyl, Ethyl, Methoxy, und/oder Ethoxy substituiertes Phenyl steht,

$R^2$ für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy steht,

m für die Zahlen 0, 1, 2 oder 3 steht,

X für Stickstoff oder eine CH-Gruppe steht und

Y für Sauerstoff, Schwefel, SO oder $SO_2$ steht,

sowie deren Säureadditions-Salze zur Bekämpfung von Krankheiten.

4. 2-(1-Chlorcyclopropyl)-1-(4-chlorphenoxy)-3-(1,2,4-triazol-1-yl)-propan-2-ol

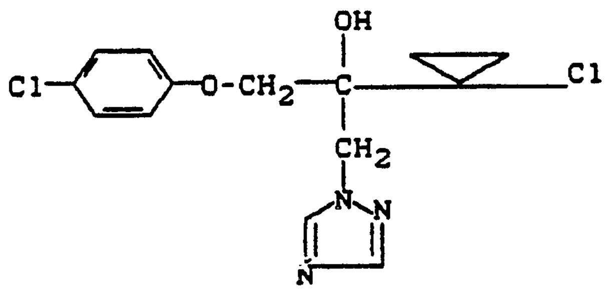

und dessen Säureadditions-Salze zur Bekämpfung von Krankheiten.

5. Hydroxyethylazolyl-Derivate nach Ansprüchen 1-4 zur Bekämpfung von Mykosen.

6. Arzneimittel, enthaltend Hydroxethylazolyl-Derivate nach Ansprüchen 1-4.

7. Antimykotische Mittel, enthaltend Hydroxyethylazolyl-Derivate nach Ansprüchen 1-4.

8. Verwendung der Hydroxyethylazolyl-Derivate nach Ansprüchen 1-4 bei der Herstellung von Arzneimitteln zur Bekämpfung von Krankheiten.

9. Verwendung der Hydroxyethylazolyl-Derivate nach Ansprüchen 1-4 bei der Herstellung von Arzneimitteln zur Bekämpfung von Mykosen.